# EUROPEAN PATENT APPLICATION

(11) **EP 0 893 122 A1**
(43) Date of publication of application: **27.01.1999**
(21) Application number: 97830394.9
(22) Date of filing: 25.07.1997
(51) Int. Cl.: A61K 31/445

(54) **Topical composition comprising thalidomide and their use in cutaneous pathologies**

(71) Applicant: Ippolito, Ferdinando, 00147 Roma (IT); Passi, Siro, 00147 Roma (IT); di Carlo, Aldo, 00147 Roma (IT)
(72) Inventor: Ippolito, Ferdinando, 00147 Roma (IT); Passi, Siro, 00147 Roma (IT); di Carlo, Aldo, 00147 Roma (IT)
(74) Representative: Sarpi, Maurizio

(57) **Abstract**

The use of thalidomide for the topical treatment of cutaneous phlogistic pathologies due to chemical, physical, bacterial, immunological causes is disclosed. A preparation in the form of gel or lipocream containing an effective concentration of thalidomide as proximate principle is proposed.

## Description

The present invention relates to the topical use of thalidomide as well as a suitable pharmaceutical preparation for such use.

As generally known, thalidomide, α-phtalimido-glutarimide acid or N-phtaloil glutamimide, is a chemical substance synthesized in 1955 in Grünenthal laboratories and used as sedative and hypnotic in medical treatments. Its empirical formula is C₁₃H₁₀N₂O₄. It is in the form of an ivory-white crystalline powder having no smell or being almost odourless, not very soluble in water, methanol, ethanol, acetone, ethyl acetate, butyl acetate, glacial acetic acid, insoluble in ethyl ether, chloroform, benzene, and very soluble in dioxane, piridine, dimethylformamide. Thalidomide consists of a mixture of the dextro-rotatory and laevorotatory isomers and, after oral exhibition, is evenly distributed in the body and prevailingly in kidney and skin rather than in liver. Tests on animals showed that the half-life is 3-4 hours, in humans being likely longer.
Thalidomide was withdrawn in 1961 because of the known teratogen effects shown by the very frequent recurrence of congenital, generally phocomelus malformations in children born from mothers who had used such a drug during pregnancy.

In 1965 Sheskin proposed the systemic use of thalidomide as sedative for patients affected by reactive lepra. As a considerable repression of the subjective and objective symptomatology not ascribable to an antimycotic activity was obtained, Sheskin emphasized the importance of such result and suggested the use of thalidomide as anti-inflammatory drug.
Such a suggestion was confirmed by other authors in a number of pathologies such as chronic erythematoid, subacute lupus erythematosus, grave aphthosis, Behçet's disease, Hebra's prurigo nodularis, actinic prurigo, graft-versus-host reaction, pyoderma gangrenosum, Melkersson-Rosenthal's macrocheilia.
Recently FDA USA has authorized the use of thalidomide in the treatment of AIDS, particularly in the ulcerative forms. The results have been extremely favourable after systemic exhibition with a posology of 100 to 300 mg per day for adults. Besides teratogen effects, other adverse effects may be related (not frequently and after long exhibition periods) to sleepiness and neurotoxic symptoms, however, reversible after reduction of the posology or suspension of the treatment.

Starting from the favourable results of the systemic treatment in a number of cutaneous, generally phlogistic pathologies, the Applicants plausibly assumed that the topical use of thalidomide could be effective also in such pathologies.
From the above statements and after a number of clinical experiments it was ascertained that the topical use of thalidomide offers an alternative to the use of corticosteroids, no cutaneous injury or vascular activity or cellular membrane injury being recognized. On the other hand, a topical use of the drug strongly reduces the teratogen effects of the same.
At the present state of art the topical use of thalidomide in dermatology has never been proposed by any Author in international field either as regard to dermatologic pathologies or to other particular aspects.

Therefore, an object of the present invention is to provide a preparation for topical use containing thalidomide as proximate principle at an effective concentration regarding cutaneous phlogistic pathologies due to chemical, physical, bacterial, immunological causes.
Such a preparation has the form of a gel or lipocream containing thalidomide at a concentration of 0.5 to 3%, preferably 1%.
On the base of the experiments such a preparation has a therapeutic activity in the following pathologies: cutaneous lupus erythematosus, pyoderma gangrenosum, photodermatitis, chronic ulcerative injuries, contact dermatitis, atopic dermatitis, psoriasis and other cutaneous phlogistic pathologic phenomena due to chemical, physical, bacterial, immunological causes.
It is self-evident that further experimental tests will allow:
- the pharmacokinetics and pharmacodynamics of thalidomide for topical use at predetermined concentrations and with a suitable excipient to be investigated thoroughly as well as the mechanism of activity thereof in the different cutaneous pathologies cited in the literature and in any other field to be experienced;
- the anti-inflammatory effectiveness over other substances of topical use (especially corticosteroids) and any topical/systemic short-term or medium-term side effects during the application to be evaluated;
- the use regarding the known anti-TNF action to be extended to the oncologic dermatologic field;
- a significant innovatory therapy not only in the dermatologic field to be acquired.

A preferred formulation for the topical use of thalidomide is described above, however, it should be understood that any other suitable excipient and vehicle may be proposed by those skilled in the art without departing from the scope of the following claims.

## Claims

1. The use of thalidomide as proximate principle in pharmaceutical preparations for topical application in the treatment of a number of cutaneous pathologies.

2. The use of thalidomide as proximate principle in pharmaceutical preparations for topical application in the treatment of the following cutaneous pathologies: lupus erythematosus, pyoderma gangrenosum, photodermatitis, chronic ulcerative injuries, contact dermatitis, atopic dermatitis, psoriasis and other cutaneous phlogistic pathologic phenomena due to chemical, physical, bacterial, immunological causes.

3. A pharmaceutical preparations for topical use in the treatment of cutaneous phlogistic pathologies, wherein it includes thalidomide at a concentration of 0.5 to 3% by weight.

4. The pharmaceutical preparations for topical use of the preceding claim, characterized in that the concentration of thalidomide is 1%.

5. The pharmaceutical preparations for topical use of the claims 3 and 4, wherein it is in the form of gel.

6. The pharmaceutical preparations for topical use of the claims 3 and 4, wherein it is in the form of lipocream.

7. The pharmaceutical preparations for topical use of the claims 3 to 6, wherein it is used in the treatment of the following pathologies: lupus erythematosus, pyoderma gangrenosum, photodermatitis, chronic ulcerative injuries, contact dermatitis, atopic dermatitis, psoriasis and other cutaneous phlogistic pathologic phenomena due to chemical, physical, bacterial, immunological causes.
